# FASCICULE DE BREVET EUROPEEN

(11) **EP 0 440 772 B1**
(45) Date de publication et mention de la délivrance du brevet: **13.03.1996**
(21) Numéro de dépôt: 90912921.5
(22) Date de dépôt: 17.08.1990
(51) Int. Cl.: C12N 15/00, C12Q 1/70

(54) **FRAGMENTS D'ACIDES NUCLEIQUES ET REACTIFS DE DIAGNOSTIC DERIVES DU GENOME DE HHV6/SIE ET PROCEDES DE DIAGNOSTIC DES INFECTIONS A HHV6**
Nukleinsäurefragmente und vom HHV6/SIE Virusgenom abgeleitete Diagnosemittel sowie Verfahren zur Diagnose von HHV6 Infektionen
NUCLEIC ACID FRAGMENTS AND DIAGNOSTIC REAGENTS DERIVED FROM THE HHV6/SIE GENOME AND PROCEDURES FOR DIAGNOSING HHV6 INFECTIONS

(30) Priorité: 18.08.1989 FR 8911016
(43) Date de publication de la demande: 14.08.1991
(60) Demande divisionnaire: 95112386.8
(73) Titulaire: INSTITUT PASTEUR, F-75724 Paris Cédex 15 (FR); UNIVERSITE PARIS VI, F-75005 Paris (FR)
(72) Inventeur: COLLANDRE, Hélène, F-75018 Paris (FR); MONTAGNIER, Luc, F-92350 Le Plessis-Robinson (FR); AGUT, Henri, F-75016 Paris (FR); BECHET, Jean-Marie, F-75005 Paris (FR)
(74) Mandataire: Ores, Irène
(86) Numéro de dépôt international: FR9000618
(87) Numéro de publication internationale: WO9102794

(56) Documents cités:
- WO-A-88/01305
- WO-A-88/09814
- Research in Virology, Volume 140, 1989, Elsevier, (Paris, FR); H. AGUT et al.: "Co-cultivation of human herpervirus 6 and HIV1 from blood lymphocytes", pages 23-26
- The Lancet, 2/9 Janvier 1988, (London, GB), S. EFSTATHIOU et al.: "DNA homology between a novel human herpesvirus (HHV-6) and human cytomegalovirus", pages 63- 64
- Leukemia, Volume 2, No. 3, Mars 1988, Williams & Wilkins, (Baltimore, US); S.F. JOSEPHS et al.: "Detection of human B-lymphotropic virus (human herpesvirus 6) sequences in B cell lymphoma tissues of three patients", pages 132-135
- Leukemia, Volume 2, No. 8, Aout 1988, Williams & Wilkins, (Baltimore, US); R.F. JARRET et al.: "Identification of human herpesvirus 6-specific DNA sequences in two patients with non-Hodgkin's lymphoma", pages 496-502
- Journal of Virology, Volume 62, No. 12, Decembre 1988, American Society for Microbiology, (Washington, DC, US); M. KISHI et al.: "A repeat sequence, GGGTTA, is shared by DNA of human herpesvirus 6 and Marek's disease virus", pages 4824-4827

## Description

La présente invention est relative à des fragments d'acides nucléiques dérivés du génome du virus HHV6, à des vecteurs contenant lesdits fragments ainsi qu'à leur utilisation dans le diagnostic des infections impliquant ce virus.

Les virus HHV6, qui sont des virus à ADN double brin, classés dans la famille des Herpes virus, ont été isolés à partir de lymphocytes de patients atteints de SIDA ou présentant des désordres lymphoprolifératifs. Ces virus sont également considérés comme étant l'agent causal de l'exanthème subit.

Différentes souches de ces virus sont connues ; la souche HBLV, qui infecte spécifiquement les lymphocytes B, a été décrite par SALAHUDIN et al. (Science 234, 396, 1986), et dans la demande PCT WO 88/01387 ; deux autres souches ont été décrites par les Inventeurs : la souche 39 TAN (AGUT et al., Res. Virol. 140, 23, 1989) et la souche SIE, qui infecte les lymphocytes T (AGUT et al., Lancet i, 712, 1988).

Le génome de la souche SIE, d'une taille d'environ 160 kb, est constitué d'ADN double brin sous forme essentiellement linéaire ; sa teneur en GC est comprise entre 38 et 40%, et il possède des séquences terminales répétitives.

La présente invention a pour but de fournir des réactifs d'acide nucléique permettant le diagnostic spécifique des infections à HHV6/SIE, et également des réactifs d'acide nucléique permettant le diagnostic général des infections impliquant une quelconque souche d'HHV6. Dans ce but, les Inventeurs ont sélectionné, cloné et caractérisé un fragment du génome viral de HHV6/SIE.

La présente invention a pour objet une séquence nucléotidique isolable du génome du virus HHV6/SIE, caractérisée en ce qu'elle est susceptible d'être obtenue par digestion avec l'enzyme Bam HI, en ce que sa taille est de 850 pb, et en ce que l'un de ses brins contient la séquence suivante, ou est capable d'hybrider en conditions stringentes avec cette séquence :

5'-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTTCTTTTGACTATTGTCTATGTCTATG
ACATTGAG...-3'
(I)

La présente invention englobe également des séquences nucléotidiques synthétiques, semi-synthétiques ou obtenues par génie génétique, dérivées des fragments d'ADN tels que définis plus haut, qu'il s'agisse de séquences d'ADN double brin contenant tout ou partie de la séquence desdits fragments, ou d'ADN ou d'ARN simple brin complémentaires de tout ou partie de l'un ou l'autre brin desdits fragments.

Un mode de réalisation préféré de la présente invention englobe en particulier les séquences oligonucléotidiques suivantes : 5'-GAXCCGACGCCXACAAACAC-3'    (II) 5'-XACCGACAXCCXXGACAXAXXAC-3'    (III) 5'-GGCXGAXXAGGAXXAAXAGGAGA-3'    (IV) dans lesquelles X = T ou U, et qui sont dérivées de la séquence (I).

Un autre mode de réalisation préféré de la présente invention comprend des séquences nucléotidiques obtenues par la méthode d'amplification PCR, en utilisant comme amorces les séquences oligonucléotidiques (II) et (III),ou bien les séquences oligonucléotidiques (III) et (IV) et comme matrice l'ADN génomique de HHV6, et dont les longueurs respectives sont de 249 et 170 pb.

Un autre mode de réalisation préféré de la présente invention comprend des séquences d'ADN recombinant, contenant tout ou partie de la séquence de l'un des fragments du génome de HHV6/SIE conformes à l'invention telles que en particulier :
- le plasmide PHC-5 qui résulte de l'insertion du fragment d'ADN de 850 pb défini plus haut, dans le plasmide PTZ 19R.

La présente invention a également pour objet des clones de cellules eucaryotes ou procaryotes transformées, contenant au moins une séquence d'ADN recombinant telle que définie plus haut.

Des clones de la souche DH5 d'Escherichia coli, transformés par les plasmides mentionnés ci-dessus ont fait l'objet d'un dépôt auprès de la Collection Nationale de Cultures de Microorganismes (CNCM), le 19 Juillet 1989 :
- le clone transformé par le plasmide pHC-5 porte le numéro de dépôt I-895.

La présente invention a également pour objet des réactifs de diagnostic, caractérisés en ce qu'ils contiennent au moins une des séquences nucléotidiques dérivées des fragments du génome du virus HHV6/SIE conformes à l'invention, telles qu'elles ont été définies précédemment, associée à au moins un moyen de détection approprié.

Lesdits réactifs peuvent être utilisés dans un très grand nombre de techniques de diagnostic basées sur la détection d'acides nucléiques par hybridation et/ou amplification ; en particulier des réactifs dérivés du fragment Bam HI de 850 pb, correspondant à une séquence conservée, peuvent permettre la détection de toutes les souches de HHV6.

La présente invention a en conséquence pour objet un procédé de diagnostic permettant soit la détection du virus HHV6/SIE, soit de façon plus générale, la détection du virus HHV6 dans un échantillon biologique, caractérisé en ce qu'il comprend une étape dans laquelle l'on met en contact l'échantillon biologique avec un réactif conforme à l'invention, et une étape dans laquelle on détecte une interaction spécifique entre ledit réactif et une ou plusieurs séquences de l'ADN dudit virus éventuellement présent dans l'échantillon biologique.

Selon un mode de mise en oeuvre préféré de ce procédé, la détection est effectuée par la méthode d'amplification PCR (amplification en chaîne par la Taq polymérase) et on utilise comme amorces et éventuellement comme sondes des séquences oligonucléotidiques dérivées des fragments d'ADN définis plus haut.

Selon une modalité préférée de ce mode de mise en oeuvre, de façon à permettre la détection de toutes les souches HHV6, on utilise comme amorces les séquences nucléotidiques (II) et (III),ou bien les séquences nucléotidiques(III) et (IV), et éventuellement comme sonde la séquence nucléotidique (IV).

La présente invention a en outre pour objet un kit pour le diagnostic des infections HHV6 et/ou HHV6/SIE, caractérisé en ce qu'il comprend au moins un réactif conforme à l'invention, associé à des moyens de mise en oeuvre d'un procédé tel que décrit plus haut.

La présente invention sera mieux comprise à l'aide du complément de description qui va suivre, qui se réfère à des exemples de préparation des réactifs conformes à l'invention, et de leur utilisation pour la détection de HHV6.

### I - PREPARATION DES FRAGMENTS ET DES MOLECULES D'ACIDE NUCLEIQUE CONFORMES A L'INVENTION

### A) ISOLEMENT ET PROPAGATION DE LA SOUCHE HHV6/SIE

La souche HHV6/SIE a été isolée à partir des lymphocytes d'une patiente atteinte d'une leucémie à HTLV-1 et séropositive pour HIV-2.

Le virus est propagé sur lymphocytes de sang périphérique.

L'isolement et la propagation de cette souche sont décrites dans la publication de AGUT et al. (Lancet i, 712, 1988).

### B) OBTENTION ET CARACTERISATION DES FRAGMENTS ET DES MOLECULES D'ACIDE NUCLEIOUE CONFORMES A L'INVENTION

### EXEMPLE 1 : Obtention et clonage d'un fragment Bam HI de 850 pb

L'ADN extrait des cellules PBL (lymphocytes de sang périphérique) infectées, est totalement digéré par l'enzyme Bam HI.

Les fragments résultants de la digestion sont séparés sur gradient de saccharose 10-30%.

Les fragments de taille inférieure à 15 kb sont insérés au site Bam HI du plasmide multifonctionnel PTZ 19R, et introduits dans les bactéries E.coli DH5.

La banque génomique ainsi obtenue est criblée par de l'ADN viral isolé du virion purifié sur gradient de saccharose 10-60% et marqué au CTP³P.

Deux recombinants ont été ainsi sélectionnés, dont un clone contenant le plasmide pHC 5, qui comporte un insert de 850 pb de l'ADN génomique de HHV6/SIE ; ce clone a été déposé le 19 Juillet 1989 auprès de la CNCM, sous le numéro d'accès I-894.

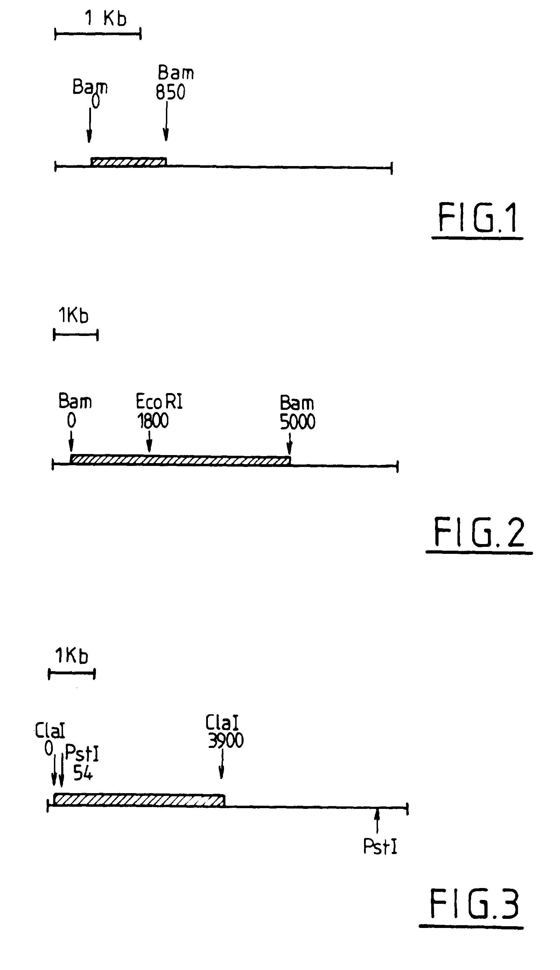

La figure 1 représente la carte de restriction du plasmide pHC 5.

La zone hachurée indique l'emplacement de l'insert de 850 pb. Cet insert ne contient aucun site de restriction pour les enzymes Hind III, Kpn I, SacI, SmaI. Cet insert a été partiellement séquencé. La séquence correspondante aux 310 premières bases est représentée par la formule (I).

### II - DETECTION DE LA PRESENCE DES VIRUS HHV6 DANS UN ECHANTILLON BIOLOGIQUE

### EXEMPLE 2 : Procédé de détection utilisant la PCR

Des Oligonucléotides synthétiques ont été obtenus à partir de la séquence (I).
- le premier (A) est constitué par la séquence (II) qui correspond aux 20 premières bases de la séquence (I).
- le deuxième (B) est constitué par la séquence (III), complémentaire des bases 227 à 249 de la séquence (I).
- le troisième (S) est constitué par la séquence (IV), et correspond aux bases 79 à 91 de la séquence (I).

Ces oligonucléotides sont utilisés dans une réaction d'amplification en chaine par la Taq polymérase.

Le milieu réactionnel contient, pour un volume total de 100 µl: 0,1 à 0,5 µM de chacune des deux amorces (A et B, ou B et S), 200µM de chacun des 4 désoxyribonucléosides triphosphates (dATP, dCTP, dGTP, dTTP), 1,5mM de MgC12, 50mM de KCl, 10mM de Tris-HCL pH 8,3, 0,1% (P/V) de gélatine, une unité de Taq DNA-polymérase (Cetus), et entre 0 et 300 ng de l'échantillon d'ADN susceptible de contenir la séquence à amplifier.

30 cycles d'amplification sont effectués, dans les conditions indiquées dans le tableau I suivant :

**TABLEAU I**

| ETAPE | TEMPERATURE | Ier CYCLE | 2/29ème CYCLE | 30ème CYCLE |
|---|---|---|---|---|
| DENATURATION | 94°C | 5mn | 1mn | 1mn |
| HYBRIDATION | 55°C | 1mn | 1mn | 1mn |
| POLYMERISATION | 72°C | 1mn | 1mn | 7mn |

Les produits de l'amplification sont analysés sur un gel d'agarose contenant lg/ml de bromure d'éthidium et peuvent être détectés par mesure de la fluorescence à 254 nm.

La spécificité de la réaction d'amplification est démontrée par détection des produits de celle-ci par la technique du "Southern blotting" : les produits de l'amplification sont transférés à partir d'un gel d'agarose sur une membrane de nylon (Zeta Probe) ; la détection est effectuée en utilisant comme sonde l'oligonucléotide S marqué au ³P.

Lorsque les oligonucléotides A et B sont utilisés comme amorces, on détecte, après 30 cycles d'amplification, un fragment de 249 pb ; lorsque les oligonucléotides B et S sont utilisés comme amorces, on détecte, après 30 cycles d'amplification , un fragment de 170 pb.

Ces deux fragments ont pu être détectés dans tous les échantillons constitués par l'ADN de cellules infectées par des virus appartenant aux souches HHV6/HBLV, HHV6/39TAN et HHV6/SIE.

La sensibilité de la détection a été déterminée en utilisant comme échantillon d'amplification soit un ADN plasmidique, dans lequel est inséré un fragment du génome du virus HHV6/SIE contenant la séquence recherchée, soit l'ADN total de cellules PBL infectées par HHV6 ; dans le premier cas, la limite inférieure de détection correspond à 0,3 fg d'ADN ; dans le deuxième, elle correspond à 5 fg d'ADN.

## Revendications (Revendications pour l'(les) Etat(s) contractant(s) suivant(s): AT, BE, CH, LI, DE, DK, FR, GB, IT, LU, NL, SE)

1. Séquence nucléotidique isolable du génome du virus HHV6/SIE, caractérisée en ce qu'elle est susceptible d'être obtenue par digestion avec 1 enzyme Bam HI, en ce que sa taille est de 850 pb, et en ce que l'un de ses brins contient la séquence suivante, ou est capable d'hybrider en conditions stringentes avec cette séquence :

5'-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTTCTTTTGACTATTGTCTATGTCTATG
ACATTGAG...-3'
(I)

2. Séquence nucléotidique caractérisée en ce qu'elle est constituée par tout ou partie d'une séquence selon la revendication 1, ou en ce qu'elle est complémentaire de tout ou partie de l'un ou l'autre brin de ladite séquence.

3. Séquence nucléotidique selon la revendication 2, caractérisée en ce qu'elle comporte ou est constituée par la séquence suivante, dans laquelle X = T ou U, ou sa séquence complémentaire :

5'-GAXCCGACGCCXACAAACAC-3'          (II)

4. Séquence nucléotidique selon la revendication 2, caractérisée en ce qu'elle comporte ou est constituée par la séquence suivante dans laquelle X = T ou U, ou sa séquence complémentaire :

5'-XACCGACAXCCXXGACAXAXXAC-3'    (III)

5. Séquence nucléotidique selon la revendication 2, caractérisée en ce qu'elle comporte ou est constituée par la séquence suivante dans laquelle X = T ou U, ou sa séquence complémentaire :

5'-GGCXGAXXAGGAXXAAXAGGAGA-3'     (IV)

6. Séquence nucléotidique selon la revendication 2, caractérisée en ce qu'elle est susceptible d'être obtenue par la méthode d'amplification PCR, en utilisant comme amorces les séquences nucléotidiques (II) et (III), et comme matrice l'ADN génomique de HHV6, et en ce que sa longueur est de 249 pb.

7. Séquence d'ADN recombinant, caractérisée en ce qu'elle comprend une séquence nucléotidique selon la revendication 2.

8. Séquence d'ADN recombinant selon la revendication 7, caractérisée en ce qu'elle est constituée par le plasmide pHC-5.

9. Clone de cellules eucaryotes ou procaryotes transformées, caractérisé en ce qu'il contient au moins une séquence d'ADN recombinant selon la revendication 7.

10. Clone selon la Revendication 9, déposé à la Collection Nationale de Cultures de Microorganismes (CNCM) sous le N°I-895 et caractérisé en ce qu'il est constitué par des cellules bactériennes de la souche *E.coli* DH5 transformées par le plasmide pHC-5.

11. Réactifs de diagnostic, caractérisés en ce qu'ils contiennent au moins une séquence nucléotidique selon l'une quelconque des revendications 1 à 7, associée à au moins un moyen de détection approprié.

12. Procédé de diagnostic de la présence du virus HHV6/SIE et/ou du virus HHV6 dans un échantillon biologique, caractérisé en ce qu'il comprend une étape dans laquelle l'on met en contact l'échantillon biologique avec un réactif selon la revendication 11, et une étape dans laquelle on détecte une interaction spécifique entre ledit réactif et une ou plusieurs séquences de l'ADN dudit virus éventuellement présent dans l'échantillon biologique.

13. Procédé selon la revendication 12, comprenant une étape d'amplification, effectuée par la méthode d'amplification PCR, caractérisé en ce qu'on utilise comme amorces et éventuellement comme sondes des séquences oligonucléotidiques dérivées de fragments d'ADN selon la revendication 1.

14. Procédé selon la revendication 13, caractérisé en ce qu'on utilise comme amorces ou bien les séquences nucléotidiques (II) et (III), ou bien les séquences nucléotidiques (III) et (IV), et éventuellement comme sonde la séquence nucléotidique (IV).

15. Kit pour le diagnostic des infections HHV6 et/ou HHV6/SIE, caractérisé en ce qu'il comprend au moins un réactif selon la revendication 11, associé à des moyens de mise en oeuvre d'un procédé selon une quelconque des revendications 12 à 14.

## Revendications (Revendications pour l'(les) Etat(s) contractant(s) suivant(s): ES)

1. Procédé d'obtention d'une séquence nucléotidique, caractérisé en ce qu'on prépare une séquence susceptible d'être isolée à partir du génome du virus HHV6/SIE par digestion avec l'enzyme Bam HI, dont la taille est de 850 pb, et dont l'un des brins contient la séquence suivante, ou est capable d'hybrider en conditions stringentes avec cette séquence :

5'-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTTCTTTTGACTATTGTCTATGTCTATG
ACATTGAG...-3'
(I)

2. Procédé selon la revendication 1, caractérisé en ce que la séquence nucléotidique préparée est constituée par tout ou partie d'une séquence selon la revendication 1, ou en ce qu'elle est complémentaire de tout ou partie de l'un ou l'autre brin de ladite séquence.

3. Procédé selon la revendication 2, caractérisé en ce que la séquence nucléotidique préparée comporte ou est constituée par la séquence suivante, dans laquelle X = T ou U, ou sa séquence complémentaire :

5'-GAXCCGACGCCXACAAACAC-3'          (II)

4. Procédé selon la revendication 2, caractérisé en ce que la séquence nucléotidique préparée comporte ou est constituée par la séquence suivante dans laquelle X = T ou U, ou sa séquence complémentaire :

5'-XACCGACAXCCXXGACAXAXXAC-3'    (III)

5. Procédé selon la revendication 2, caractérisé en ce que la séquence nucléotidique préparée comporte ou est constituée par la séquence suivante dans laquelle X = T ou U, ou sa séquence complémentaire :

5'-GGCXGAXXAGGAXXAAXAGGAGA-3'    (IV)

6. Procédé d'obtention d'une séquence nucléotidique tel que définie dans la revendication 2, caractérisé en ce que l'on met en oeuvre la méthode d'amplification PCR, en utilisant comme amorces les séquences nucléotidiques (II) et (III), et comme matrice l'ADN génomique de HHV6, et en ce que sa longueur est de 249 pb.

7. Procédé d'obtention d'une séquence d'ADN recombinant, caractérisé en ce que ladite séquence d'ADN comprend une séquence nucléotidique selon la revendication 2.

8. Procédé selon la revendication 7, caractérisée en ce que ladite séquence d'ADN recombinant est constituée par le plasmide pHC-5.

9. Procédé d'obtention d'un clone de cellules eucaryotes ou procaryotes transformées, caractérisé en ce que lesdites cellules sont transformées par au moins une séquence d'ADN recombinant selon la revendication 7.

10. Procédé d'obtention d'un clone de cellules eucaryotes ou procaryotes transformées, selon la Revendication 9, caractérisé en ce que ledit clone est constitué par des cellules bactériennes de la souche *E.coli* DH5 transformées par le plasmide pHC-5, et a été déposé à la Collection Nationale de Cultures de Microorganismes (CNCM) sous le N°I-895.

11. Procédé d'obtention de réactifs de diagnostic, caractérisé en ce que l'on associe au moins une séquence nucléotidique selon l'une quelconque des revendications 1 à 7, à au moins un moyen de détection approprié.

12. Procédé de diagnostic de la présence du virus HHV6/SIE et/ou du virus HHV6 dans un échantillon biologique, caractérisé en ce qu'il comprend une étape dans laquelle l'on met en contact l'échantillon biologique avec un réactif obtenu par le procédé selon la revendication 11, et une étape dans laquelle on détecte une interaction spécifique entre ledit réactif et une ou plusieurs séquences de l'ADN dudit virus éventuellement présent dans l'échantillon biologique.

13. Procédé selon la revendication 12, comprenant une étape d'amplification, effectuée par la méthode d'amplification PCR, caractérisé en ce qu'on utilise comme amorces et éventuellement comme sondes des séquences oligonucléotidiques dérivées de fragments d'ADN selon la revendication 1.

14. Procédé selon la revendication 13, caractérisé en ce qu'on utilise comme amorces ou bien les séquences nucléotidiques (II) et (III), ou bien les séquences nucléotidiques (III) et (IV), et éventuellement comme sonde la séquence nucléotidique (IV).

15. Procédé d'obtention d'un kit pour le diagnostic des infections HHV6 et/ou HHV6/SIE, caractérisé en ce que l'on associe au moins un réactif obtenu par le procédé selon la revendication 11, à des moyens de mise en oeuvre d'un procédé selon une quelconque des revendications 12 à 14.

## Patentansprüche (Patentansprüche für folgende(n) Vertragsstaat(en): AT, BE, CH, DE, DK, FR, GB, IT, LI, LU, NL, SE)

1. Nucleotidsequenz isolierbar aus dem Genom des Virus HHv6/SIE, gekennzeichnet dadurch, daß sie erhältlich ist durch Verdau mit dem Enzym Bam HI, durch ihre Größe von 850 bp, und dadurch, daß einer ihrer Stränge die nachfolgende Sequenz enthält oder in der Lage ist, unter stringenten Bedingungen mit dieser Sequenz zu hybridisieren:

5'-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTTCTTTTGACTATTGTCTATGTCTATG
ACATTGAG...-3'
(I)

2. Nucleotidsequenz, dadurch gekennzeichnet, daß sie aus der gesamten oder einem Teil einer Sequenz nach Anspruch 1 besteht, oder dadurch, daß sie komplementär ist zum gesamten oder einem Teil des einen oder des anderen Stranges dieser Sequenz.

3. Nucleotidsequenz nach Anspruch 2, dadurch gekennzeichnet, daß sie die folgende Sequenz, in der X = T oder U ist, oder deren komplementäre Sequenz enthält oder aus dieser besteht:

5'-GAXCCGACGCCXACAAACAC-3'    (II)

4. Nucleotidsequenz nach Anspruch 2, dadurch gekennzeichnet, daß sie die folgende Sequenz, in der X = T oder U ist, oder deren komplementäre Sequenz enthält oder aus dieser besteht:

5'-XACCGACAXCCXXGACAXAXXAC-3'     (III)

5. Nucleotidsequenz nach Anspruch 2, dadurch gekennzeichnet, daß sie die folgende Sequenz, in der X = T oder U ist, oder deren komplementäre Sequenz enthält oder aus dieser besteht:

5'-GGCXGAXXAGGAXXAAXAGGAGA-3'     (IV)

6. Nucleotidsequenz nach Anspruch 2, dadurch gekennzeichnet, daß sie erhältlich ist durch die PCR-Amplifikationsmethode unter Verwendung der Nucleotidsequenzen (II) und (III) als Startermoleküle und der genomischen DNA von HHV6 als Matrize, und dadurch, daß ihre Länge 249 bp beträgt.

7. Rekombinante DNA-Sequenz, dadurch gekennzeichnet, daß sie eine Nucleotidsequenz nach Anspruch 2 enthält.

8. Rekombinante DNA-Sequenz nach Anspruch 7, dadurch gekennzeichnet, daß sie das Plasmid pHC-5 ist.

9. Clon transformierter eukaryontischer oder prokaryontischer Zellen, dadurch gekennzeichnet, daß er mindestens eine rekombinante DNA-Sequenz nach Anspruch 7 enthält.

10. Clon nach Anspruch 9, hinterlegt bei der "Collection Nationale de Cultures de Microorganismes" (CNCM) unter der Nr. I-895 und dadurch gekennzeichnet, daß er aus bakteriellen Zellen des E. coli-Stammes DH5 besteht, die mit dem Plasmid pHC-5 transformiert sind.

11. Diagnostische Mittel, dadurch gekennzeichnet, daß sie mindestens eine Nucleotidsequenz nach einem der Ansprüche 1 bis 7 enthalten, in Verbindung mit mindestens einem geeigneten Nachweismittel.

12. Verfahren zur Diagnose der Anwesenheit des Virus HHV6/SIE und/oder des Virus HHV6 in einer biologischen Probe, dadurch gekennzeichnet, daß es einen Schritt umfaßt, in dem die biologische Probe mit einem Mittel nach Anspruch 11 in Kontakt gebracht wird, und einen Schritt, in dem eine spezifische Interaktion nachgewiesen wird, zwischen diesem Mittel und einer oder mehreren Sequenzen der DNA des Virus, die gegebenenfalls in der biologischen Probe vorhanden ist.

13. Verfahren nach Anspruch 12, umfassend einen Amplifikationsschritt, ausgeführt mittels der PCR-Amplifikationsmethode, dadurch gekennzeichnet, daß als Startermoleküle und gegebenenfalls als Sonden Oligonucleotidsequenzen verwendet werden, die von DNA-Fragmenten nach Anspruch 1 abgeleitet sind.

14. Verfahren nach Anspruch 13, dadurch gekennzeichnet, daß als Startermoleküle entweder die Nucleotidsequenzen (II) und (III) oder die Nucleotidsequenzen (III) und (IV) verwendet werden, und gegebenenfalls als Sonde die Nucleotidsequenz (IV).

15. Testbesteck zur Diagnose von HHV6- und/oder HHV6/SIE-Infektionen, dadurch gekennzeichnet, daß es mindestens ein Mittel nach Anspruch 11 enthält, in Verbindung mit Mitteln zur Ausführung eines Verfahrens nach einem der Ansprüche 12 bis 14.

## Patentansprüche (Patentansprüche für folgende(n) Vertragsstaat(en): ES)

1. Verfahren zum Erhalt einer Nucleotidsequenz, dadurch gekennzeichnet, daß eine Sequenz hergestellt wird, die isolierbar ist ausgehend von dem Genom des Virus HHV6/SIE durch Verdau mit dem Enzym Bam HI, deren Größe 850 bp beträgt und deren einer Strang die folgende Sequenz enthält oder in der Lage ist, mit dieser Sequenz unter stringenten Bedingungen zu hybridisieren:

5'-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTCTTTTGACTATTGTCTATGTCTATG
ACATTGAG...-3'
(I)

2. Verfahren nach Anspruch 1, dadurch gekennzeichnet, daß die hergestellte Nucleotidsequenz aus der gesamten oder einem Teil einer Sequenz nach Anspruch 1 besteht, oder dadurch, daß sie komplementär ist zum gesamten oder einem Teil des einen oder anderen Stranges dieser Sequenz.

3. Verfahren nach Anspruch 2, dadurch gekennzeichnet, daß die hergestellte Nucleotidsequenz die folgende Sequenz, in der X = T oder U ist, oder deren komplentäre Sequenz enthält oder aus dieser besteht:

5'-GAXCCGACGCCXACAAACAC-3'    (II)

4. Verfahren nach Anspruch 2, dadurch gekennzeichnet, daß die hergestellte Nucleotidsequenz die folgende Sequenz, in der X = T oder U ist, oder deren komplementäre Sequenz enthält oder aus dieser besteht:

5'-XACCGACAXCCXXGACAXAXXAC-3'    (III)

5. Verfahren nach Anspruch 2, dadurch gekennzeichnet, daß die hergestellte Nucleotidsequenz die folgende Sequenz, in der X = T oder U ist, oder deren komplementäre Sequenz enthält oder aus dieser besteht:

5'-GGCXGAXXAGGAXXAAXAGGAGA-3'     (IV)

6. Verfahren zum Erhalt einer Nucleotidsequenz wie in Anspruch 2 definiert, dadurch gekennzeichnet, daß die PCR-Amplifikationsmethode durchgeführt wird unter Verwendung der Nucleotidsequenzen (II) und (III) als Startermoleküle und der genomischen DNA von HHV6 als Matrize, und dadurch, daß ihre Länge 249 bp beträgt.

7. Verfahren zum Erhalt einer rekombinanten DNA-Sequenz, dadurch gekennzeichnet, daß diese DNA-Sequenz eine Nucleotidsequenz nach Anspruch 2 enthält.

8. Verfahren nach Anspruch 7, dadurch gekennzeichnet, daß die rekombinante DNA-Sequenz das Plasmid pHC-5 ist.

9. Verfahren zum Erhalt eines Clons transformierter eukaryontischer oder prokaryontischer Zellen, dadurch gekennzeichnet, daß die Zellen mit mindestens einer rekombinanten DNA-Sequenz nach Anspruch 7 transformiert sind.

10. Verfahren zum Erhalt eines Clons transformierter eukaryontischer oder prokaryontischer Zellen nach Anspruch 9, dadurch gekennzeichnet, daß der Clon aus bakteriellen Zellen des E. coli Stammes DH5 besteht, die mit dem Plasmid pHC-5 transformiert sind, und bei der "Collection Nationale de Cultures de Microorganismes" (CNCM) unter der Nr. I-895 hinterlegt ist.

11. Verfahren zum Erhalt diagnostischer Mittel, dadurch gekennzeichnet, daß mindestens eine Nucleotidsequenz nach einem der Ansprüche 1 bis 7 in Verbindung gebracht wird mit mindestens einem geeigneten Nachweismittel.

12. Verfahren zur Diagnose der Anwesenheit des Virus HHV6/SIE und/oder des Virus HHV6 in einer biologischen Probe, dadurch gekennzeichnet, daß es einen Schritt umfaßt, in dem die biologische Probe mit einem Mittel in Kontakt gebracht wird, das durch das Verfahren nach Anspruch 11 erhalten wurde, und einen Schritt, in dem eine spezifische Interaktion nachgewiesen wird, zwischen dem Mittel und einer oder mehreren Sequenzen der DNA des Virus, die gegebenenfalls in der biologischen Probe vorhanden ist.

13. Verfahren nach Anspruch 12, umfassend einen Amplifikationsschritt, ausgeführt mittels der PCR-Amplifikationsmethode, dadurch gekennzeichnet, daß als Startermoleküle und gegebenenfalls als Sonden Oligonucleotidsequenzen verwendet werden, die von DNA-Fragmenten nach Anspruch 1 abgeleitet sind.

14. Verfahren nach Anspruch 13, dadurch gekennzeichnet, daß als Startermoleküle entweder die Nucleotidsequenzen (II) und (III) oder die Nucleotidsequenzen (III) und (IV) verwendet werden, und gegebenenfalls als Sonde die Nucleotidsequenz (IV).

15. Verfahren zum Erhalt eines Testbestecks zur Diagnose von HHV6- und/oder HHV6/SIE-Infektionen, dadurch gekennzeichnet, daß mindestens ein Mittel, das durch das Verfahren nach Anspruch 11 erhalten wurde, mit Mitteln zur Ausführung eines Verfahrens nach einem der Ansprüche 12 bis 14 in Verbindung gebracht wird.

## Claims (Claims for the following Contracting State(s): AT, BE, CH, DE, DK, FR, GB, IT, LI, LU, NL, SE)

1. Nucleotide sequence which can be isolated from the genome of the HHV6/SIE virus, characterised in that it is able to be obtained by digestion with the enzyme Bam HI, in that its size is 850 bp and in that one of its strands contains the following sequence or is capable of hybridising under stringent conditions with this sequence:

5′-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTTCTTTTGACTATTGTCTATGTCTATG
ACATTGAG...-3′.
(I)

2. Nucleotide sequence, characterised in that it consists of all or part of a sequence according to Claim 1, or in that it is complementary to all or part of one or other strand of the said sequence.

3. Nucleotide sequence according to Claim 2, characterised in that it contains or consists of the following sequence, in which X = T or U, or its complementary sequence:

5'-GAXCCGACGCCXACAAACAC-3'          (II)

4. Nucleotide sequence according to Claim 2, characterised in that it contains or consists of the following sequence in which X = T or U, or its complementary sequence:

5'-XACCGACAXCCXXGACAXAXXAC-3'          (III)

5. Nucleotide sequence according to Claim 2, characterised in that it contains or consists of the following sequence in which X = T or U, or its complementary sequence:

5'-GGCXGAXXAGGAXXAAXAGGAGA-3'    (IV)

6. Nucleotide sequence according to Claim 2, characterised in that it is capable of being obtained by the PCR amplification method, using the nucleotide sequences (II) and (III) as primers and the HHV6 genome DNA as template, and in that its length is 249 bp.

7. Recombinant DNA sequence, characterised in that it contains a nucleotide sequence according to Claim 2.

8. Recombinant DNA sequence according to Claim 7, characterised in that it consists of the plasmid pHC-5.

9. Clone of transformed eucaryotic or procaryotic cells, characterised in that it contains at least one recombinant DNA sequence according to Claim 7.

10. Clone according to Claim 9, filed with the Collection Nationale de Cultures de Microorganismes (CNCM; National Collection of Microorganism Cultures) under the No. I-895 and characterised in that it consists of bacterial cells of the *E. coli* DH5 strain transformed by the plasmid pHC-5.

11. Diagnostic reagents, characterised in that they contain at least one nucleotide sequence according to any one of Claims 1 to 7 in combination with at least one appropriate detection means.

12. Procedure for diagnosing the presence of the HHV6/SIE virus and/or the HHV6 virus in a biological sample, characterised in that it comprises a step in which the biological sample is brought into contact with a reagent according to Claim 11, and a step in which a specific interaction is detected between the said reagent and one or more DNA sequences of the said virus which may be present in the biological sample.

13. Procedure according to Claim 12, comprising an amplification step, carried out by the PCR amplification method, characterised in that oligonucleotide sequences derived from DNA fragments according to Claim 1 are used as primers and, where appropriate, as probes.

14. Procedure according to Claim 13, characterised in that either the nucleotide sequences (II) and (III) or the nucleotide sequences (III) and (IV) are used as primers and the nucleotide sequence (IV) is used, where appropriate, as probe.

15. Kit for diagnosing HHV6 and/or HHV6/SIE infections, characterised in that it comprises at least one reagent according to Claim 11 in combination with means for carrying out a procedure according to any one of Claims 12 to 14.

## Claims (Claims for the following Contracting State(s): ES)

1. Procedure for obtaining a nucleotide sequence, characterised in that a sequence which can be isolated from the genome of the HHV6/SIE virus by digestion with the enzyme Bam HI is prepared, the size of which is 850 bp, and one of the strands of which contains the following sequence or is capable of hybridising under stringent conditions with this sequence:

5'-GATCCGACGCCTACAAACACGTCGATAATTTGATATAATAAGTGGAAAACTCTG
TTCCAGAGAAAGGGTGTTGCGAAGGGCTGATTAGGATTAATAGGAGAATCTTGTAAG
TATATGGTCCAGTTGAAATTAGATTTCATTATAGGAAAAGATTTGAGATCGTGAAAT
AAAGATTGAAATTGTGATCGTAGTTCATAAAAACTTGGAATGAGGTCACTTCTGTTG
CGTAATATGTCAAGGATGTCGGTACTTTTTTTCTTTTGACTATTGTCTATGTCTATG
ACATTGAG...-3'.
(I)

2. Procedure according to Claim 1, characterised in that the nucleotide sequence prepared consists of all or part of a sequence according to Claim 1, or in that it is complementary to all or part of one or other strand of the said sequence.

3. Procedure according to Claim 2, characterised in that the nucleotide sequence prepared contains or consists of the following sequence, in which X = T or U, or its complementary sequence:

5'-GAXCCGACGCCXACAAACAC-3'     (II)

4. Procedure according to Claim 2, characterised in that the nucleotide sequence prepared contains or consists of the following sequence in which X = T or U, or its complementary sequence:

5'-XACCGACAXCCXXGACAXAXXAC-3'    (III)

5. Procedure according to Claim 2, characterised in that the nucleotide sequence prepared contains or consists of the following sequence in which X = T or U, or its complementary sequence:

5'-GGCXGAXXAGGAXXAAXAGGAGA-3'    (IV)

6. Procedure for obtaining a nucleotide sequence as defined in Claim 2, characterised in that use is made of the PCR amplification method, using the nucleotide sequences (II) and (III) as primers and the HHV6 genome DNA as template, and in that its length is 249 bp.

7. Procedure for obtaining a recombinant DNA sequence, characterised in that the said DNA sequence contains a nucleotide sequence according to Claim 2.

8. Procedure according to Claim 7, characterised in that the said recombinant DNA sequence consists of the plasmid pHC-5.

9. Procedure for obtaining a clone of transformed eucaryotic or procaryotic cells, characterised in that the said cells are transformed by at least one recombinant DNA sequence according to Claim 7.

10. Procedure for obtaining a clone of transformed eucaryotic or procaryotic cells according to Claim 9, characterised in that the said clone consists of bacterial cells of the strain *E.coli* DH5 transformed by the plasmid pHC-5 and has been filed with the Collection Nationale de Cultures de Microorganismes (CNCM; National Collection of Microorganism Cultures) under the No. I-895.

11. Procedure for obtaining diagnostic reagents, characterised in that at least one nucleotide sequence according to any one of Claims 1 to 7 is combined with at least one appropriate detection means.

12. Procedure for diagnosing the presence of the HHV6/SIE virus and/or the HHV6 virus in a biological sample, characterised in that it comprises a step in which the biological sample is brought into contact with a reagent obtained by the procedure according to Claim 11, and a step in which a specific interaction is detected between the said reagent and one or more DNA sequences of the said virus which may be present in the biological sample.

13. Procedure according to Claim 12, comprising an amplification step, carried out by the PCR amplification method, characterised in that oligonucleotide sequences derived from DNA fragments according to Claim 1 are used as primers and, where appropriate, as probes.

14. Procedure according to Claim 13, characterised in that either the nucleotide sequences (II) and (III) or the nucleotide sequences (III) and (IV) are used as primers and the nucleotide sequence (IV) is used, where appropriate, as probe.

15. Procedure for obtaining a kit for diagnosing HHV6 and/or HHV6/SIE infections, characterised in that at least one reagent obtained by the procedure according to Claim 11 is combined with means for carrying out a procedure according to any one of Claims 12 to 14.
